# EUROPEAN PATENT APPLICATION

(11) **EP 4 044 191 A1**
(43) Date of publication of application: **17.08.2022**
(21) Application number: 20873826.0
(22) Date of filing: 07.10.2020
(51) Int. Cl.: G16H 20/00, G16H 20/10, G16H 20/40

(54) **INSTRUCTION DOCUMENT CREATING SYSTEM**

(30) Priority: 09.10.2019 JP 2019186296
(71) Applicant: Iryou Jyouhou Gijyutu Kenkyusho Corporation, Yame-gun, Fukuoka 834-0102 (JP)
(72) Inventor: Himeno Shinkichi, Yame-gun Fukuoka 834-0102 (JP)
(74) Representative: Gulde & Partner
(86) International application number: PCT/JP2020/037928
(87) International publication number: WO 2021/070839

(57) **Abstract**

[Problem] To prevent side effects or eliminate description omission of indications by automatically checking related check items when creating an instruction document for each medical practice.

[Solving Means] An instruction document creating system for creating an instruction document mainly in the medical field includes (i) instruction item-specific rules recording means configured to record rules to be observed with respect to each of instruction items in the instruction document, (ii) rule consistency check means configured to perform checks on consistency between the instruction items in the instruction document and the rules recorded in the instruction item-specific rules recording means on the basis of the rules, and (iii) check log document creating means configured to create results of the rule consistency checks as a document related to the instruction document.

## Description

### Technical Field

The present invention relates to an instruction document creating system for creating an instruction document (order) mainly in the medical field. More specifically, the present invention relates to an instruction document creating system that creates an instruction document that is safe and is less likely to cause assessments of medical fees by checking medical safety or the consistency to rules on medical fee billing.

### BACKGROUND ART

In medical sites and the like, various instruction documents on drug prescription, tests, rehabilitation, and the like are created, and drug formulation, tests, rehabilitation, and the like are performed on the basis of the instruction documents.

While the effects or sharpness of drugs, treatment, tests, and the like have been improved, drugs and the like having serious side effects have been increased. Also, the current society is an aging society. For this reason, it is inevitable to carefully cope with patients having multiple severe diseases in terms of effects or side effect prevention.

Also, to suppress an increase in the medical expenditure, the medical fee for medical practice, such as a test and drug prescription, is assessed (reduced or not paid) unless the practice strictly observes the medical fee rules. Such cases are being increased sharply and are affecting the management of medical institutions.

Background art literature relating to the present application includes the following.

### Citation List

### Patent Literature

Patent Literature 1: Japanese Unexamined Patent Application Publication No. 2019-101491
Patent Literature 2: Japanese Unexamined Patent Application Publication No. 2009-146345 SUMMARY OF INVENTION

### Technical Problem

To protect the medical safety of patients, drugs, tests, and treatment each have many pre-check items. For example, a certain drug has many check items including: patients having reduced renal function or patients with hepatitis contraindicate the drug; the drug should carefully be administered to elderly persons; and side effects need to be checked in a monthly blood test.

Since each drug has such check items, sufficiently checking even a single prescription requires not only the prescription doctor but also the pharmacist, nurses, and the like to take much time and effort. If the pharmacist or the like becomes aware that any check item has been omitted, they need to inquire of the prescription doctor about the omitted check item. However, in many cases, it is difficult to smoothly consult with the doctor, who is busy.

With respect to the discretion of the doctor, it is often uncertain whether the doctor has dared to prescribe the drug after checking the check item or has overlooked the check item. Under the current circumstances, much time and effort is being consumed to make inquiries about such omitted check items.

Indications and usage/dosage are determined with respect to every medical practice. If any medical practice is not provided with indication names or is performed so as to deviate from the usage/dosage thereof, the entire medical fee for the medical practice is assessed, resulting in a serious blow to the management. Before submitting medical fee bills, the staff of the medical affairs department has to check the bills for a long time, resulting in much overtime work.

The present invention has been made to solve the above background art problems, and an object thereof is to prevent side effects or eliminate the description omission of indications by automatically checking related check items when creating an instruction document for each medical practice. Another object is to reduce unnecessary verification work done between the prescription doctor and the pharmacist or the like by creating a document that records judgments or additional practice made by the prescription doctor with respect to check items on which a warning has been issued (check log document). Yet another object is to provide an instruction document creating system that is able to minimize the assessment of medical fees by not only observing required usage/dosage but also describing indication names when creating an instruction document.

### Solution to Problem

As means for accomplishing the above objects, an instruction document creating system for creating an instruction document mainly in a medical field according to claim 1 includes (i) instruction item-specific rules recording means configured to record rules to be observed with respect to each of instruction items in the instruction document, (ii) rule consistency check means configured to perform checks on consistency between the instruction items in the instruction document and the rules recorded in the instruction item-specific rules recording means on the basis of the rules, and (iii) check log document creating means configured to create results of the rule consistency checks as a document related to the instruction document.

According to the instruction document creating system of claim 2, in the instruction document creating system of claim 1, the rule consistency check means includes information-required-to-check-consistency request means configured to request information required to check the consistency from medical record documents of a patient involved.

According to the instruction document creating system of claim 3, in the instruction document creating system of claim 1 or 2, the rule consistency check means includes after-consistency checks additional information write means configured to request a write, to the medical record documents of the patient involved, of an additional disease name, an additional finding, and an additional instruction document that have occurred in a process of the consistency checks.

According to the instruction document creating system of claim 4, in the instruction document creating system of any one of claims claim 1 to 3, the rule consistency check means includes after-consistency confirmation additional information write means configured to, after an additional disease name, an additional finding, and an additional instruction document that have not been confirmed in a process of the consistency checks are confirmed, request a write of the additional disease name, the additional finding, and the additional instruction document to the medical record documents of the patient involved.

According to the instruction document creating system of claim 5, in the instruction document creating system of any one of claims 1 to 4, the instruction item-specific rules recording means includes multiple instruction items-specific rules recording means configured to record rules corresponding to multiple instruction items together.

### Advantageous Effects of Invention

The instruction document creating system for creating an instruction document mainly in a medical field according to claim 1 includes the instruction item-specific rules recording means and thus records rules to be observed with respect to each of instruction items in the instruction document.

The instruction document creating system of claim 1 includes the rule consistency check means and thus performs checks on consistency between the instruction items in the instruction document and the rules recorded in the instruction item-specific rules recording means on the basis of the rules.

The instruction document creating system of claim 1 includes the check log document creating means and thus creates results of the rule consistency checks as a document related to the instruction document.

The instruction document creating system of claim 2 includes the information-required-to-check-consistency request means and thus requests information required to check the consistency from medical record documents of a patient involved.

The instruction document creating system of claim 3 includes the after-consistency checks additional information write means and thus requests a write, to the medical record documents of the patient involved, of an additional disease name, an additional finding, and an additional instruction document that have occurred in a process of the consistency checks.

The instruction document creating system of claim 4 includes the after-consistency confirmation additional information write means and thus, after an additional disease name, an additional finding, and an additional instruction document that have not been confirmed in a process of the consistency checks are confirmed, requests a write of the additional disease name, the additional finding, and the additional instruction document to the medical record documents of the patient involved.

The instruction document creating system of claim 5 includes the multiple instruction items-specific rules recording means and thus records rules corresponding to multiple instruction items together.

### BRIEF DESCRIPTION OF THE DRAWINGS

FIG. 1 shows an example of an instruction item (here, Loxonin tablets) and descriptions in the instruction item-specific rules recording means of the instruction item in a drug prescription (drug prescription instruction document).
FIG. 2 shows an example of an instruction item (here, lumbar MRI) and descriptions in the instruction item-specific rules recording means of the instruction item in a radiographic examination prescription (an instruction document for prescribing Xp, MRI, or the like).

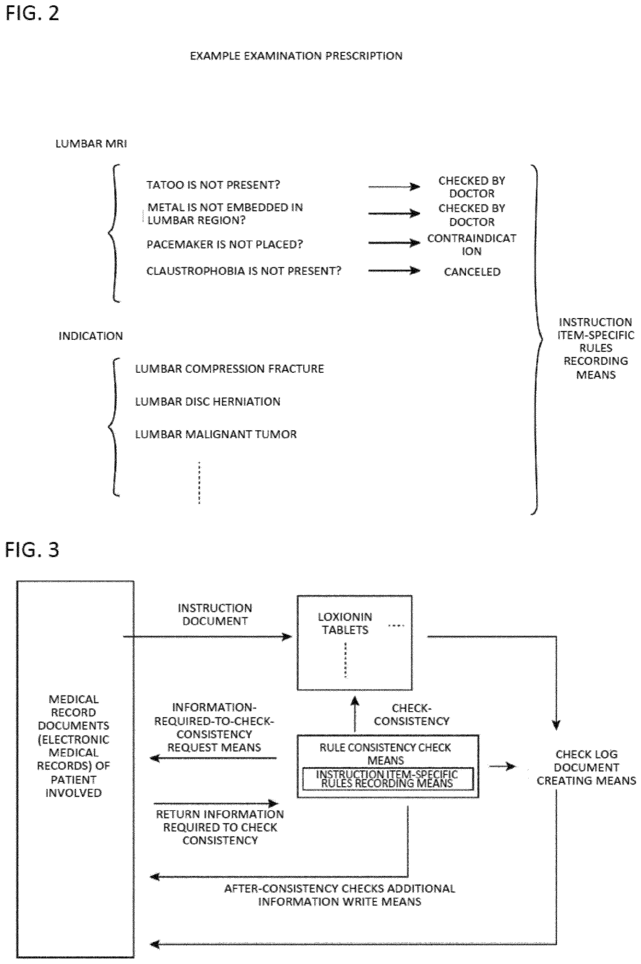

FIG. 3 is a diagram showing an outline of the present invention. The rule consistency of each of instruction items described in an instruction document issued from medical record documents (electronic medical records) of the patient involved is checked on the basis of the corresponding instruction item-specific rules recording means (rule consistency check means). Information required to judge the consistency is requested from the electronic medical records (information-required-to-check-consistency request means). The rule consistency is checked using a response to the request, and a write of additional disease names, additional findings, and additional instruction documents to the medical record documents of the patient is requested (after-consistency checks additional information write means). The above process (check log), which is a rule check process related to the instruction document, is recorded as a document related to the instruction document (check log document creating means).
FIG. 4 shows an example check log document.

### DESCRIPTION OF EMBODIMENTS

The present application is a system that is run using a computer.

This computer includes an input device (mouse, keyboard, etc.), an output device (monitor, printer, etc.), a storage device (memory, hard disk), a computing device (CPU), a controller (CPU), and the like, as well as includes a program for performing the means (functions) of the present application.

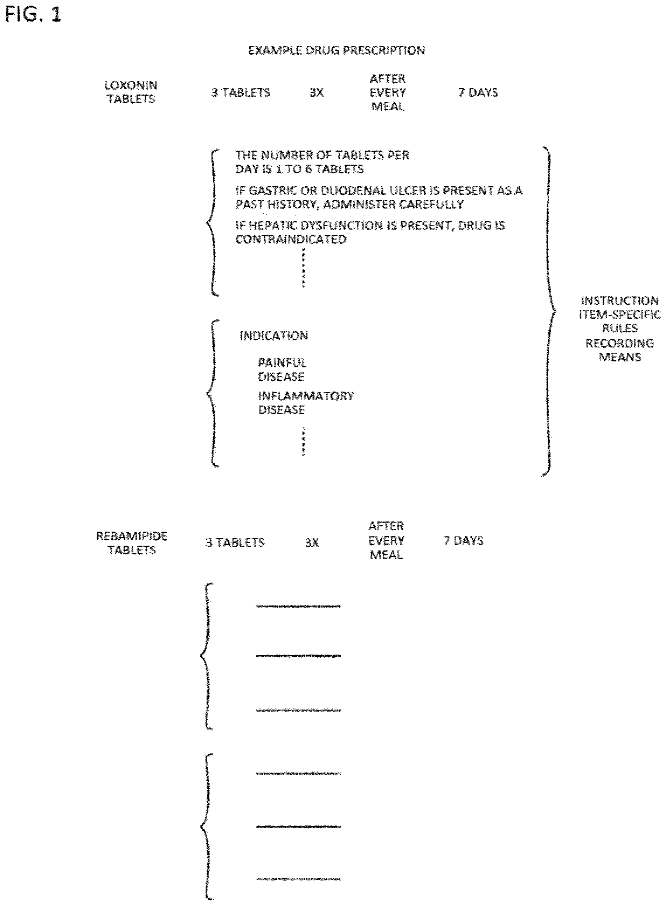

FIG. 1 shows an example of an instruction item (here, Loxonin tablets) and descriptions in the instruction item-specific rules recording means of the instruction item in a drug prescription (drug prescription instruction document).

The instruction item-specific rules recording means is recording rules for ensuring safety, such as "the number of tablets per day falls within the range?" "gastric or duodenal ulcer is not present as a past history? if present, administer carefully," "hepatic dysfunction is not present? if present, the drug is contraindicated."

The instruction item-specific rules recording means is also recording a list of indication names of this drug. The indication names may be direct ones, such as "lumbar disc herniation." The indication names may also be group names representing generic disease names, such as "painful disease," and a disease name may be selected from the applicable group name when necessary.

The rules including the indication names may be separately recorded for each instruction item, or may be recorded together in different files or the like as necessary.

It is also useful to previously convert the instruction items into objects by integrating the format and rules, to drop any object on an instruction document, and to guide a description according to the format or to execute a script based on the rules set on the item.

Business rule management systems are attracting attention in recent years. A business rule management system previously describes a great number of rules on business in a rule description language, scans the rules when necessary, and applies the rules sequentially. While the rules may be described for each instruction item as shown in FIG. 1, a great number of rules to which the application target (here, "Loxonin tablets") has been added may be managed centrally or in multiple groups using a rule-based engine (multiple instruction items-specific rule recording means).

FIG. 2 shows an example of an instruction item (here, lumbar MRI) and descriptions in the instruction item-specific rules recording means of the instruction item in a radiographic examination prescription (an instruction document for prescribing Xp, MRI, or the like).

If a tattoo is present, a burn may occur.

While the blue color of carbon is relatively safe, a coloring matter including a red iron content is more likely to cause a burn. In any case, it is necessary to consult with the instruction doctor.

If a metal is embedded at the imaging site, magnetic lines of force may cause fever or image distortion. Again, it is necessary to consult with the instruction doctor.

If a pacemaker is placed in the body, it is more likely to malfunction and therefore is a contraindication.

If claustrophobia is present as a past history, the examination is more likely to be non-executable and therefore is canceled.

If there is a list of indication names on an insurance medical fee bill, it is necessary to select an indication name after imaging or select an indication name that the examination has been conducted on suspicion of the patient having (suspected disease name), to record the selected indication name on an electronic health record, and thus to reflect it on a medical fee bill so that the medical fee is not assessed.

FIG. 3 is a diagram showing an outline of the present invention.

The rule consistency of each of instruction items described in an instruction document issued from medical record documents (electronic medical records) of the patient involved is checked on the basis of the corresponding instruction item-specific rules recording means (rule consistency check means).

Information required to judge the consistency, for example, blood test data, already registered disease names, past histories, the presence or absence of particular symptoms or findings, or the like, is requested from the electronic medical records (information-required-to-check-consistency request means).

The rule consistency is checked using a response to the request.

The disease names and symptoms or findings added in this process and an instruction document for a test or the like added as needed are requested to be written to the medical record documents of the patient (after-consistency checks additional information write means).

The above process (check log), which is a rule check process with respect to the instruction document, is recorded as a document related to the instruction document (check log document creating means).

If the results of the additional test and the like cannot be obtained soon, the rule consistency check is temporarily put on hold. The instruction itself becomes "tentatively executed," or "execution is put on hold until the results are obtained." After the results are obtained, the instruction is executed or stopped and disease names and symptoms or findings obtained after the checks are added to the electronic medical records (after-consistency checks additional information write means).

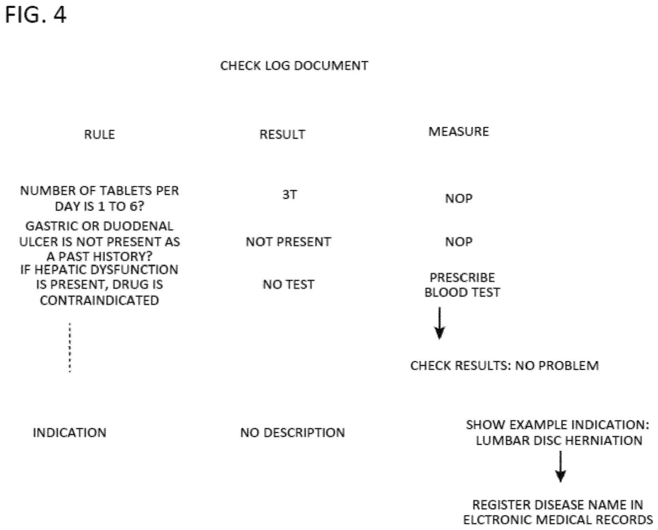

FIG. 4 shows an example check log document.

The number of tablets fell within the rule and therefore there was no need to make a reaction (NOP).

Also, gastric or duodenal ulcer was not present as a past history and therefore a NOP was issued. With respect to the rule stating that if hepatic dysfunction is present, drug is contraindicated, the electronic medical records were consulted for data on hepatic function based on a blood test (information-required-to-check-consistency request means). However, a blood test had yet to be conducted. For this reason, the prescription was tentatively performed, an instruction document for a blood test was issued, and an obtained result was checked. As a result, no problem was found. For this reason, a description to that effect was recorded in the article of the doctor, and the prescription of the drug was continued.

Although the existing registered disease names were consulted for the indication names of the drug, the indication names were not present. For this reason, "lumbar disc herniation" was selected from the indication name list, and the disease name was registered in the electronic medical records (after-consistency checks additional information write means).

By creating the check log document as a document related to the instruction document, the flow of thought as to what the prescription doctor has thought with respect to the rule-based warning about the instruction document and how they have coped with the warning is recorded. This prevents the prescription doctor from overlooking the rules based on the safety standards, leading to an improvement in safety.

The judgments of the prescription doctor with respect to the rules on which the doctor can exercise discretion (for example, with respect to hepatic dysfunction, what value or greater becomes a contraindication?, etc.) are clarified. Referring to these judgments makes it unnecessary for the pharmacist, nurses, or the like to make a great number of inquiries about questions, which have been made so far. This facilitates the work of not only the doctor but also the staff members.

Also, the judgments made at the discretion of the doctor are explicitly recorded. Thus, in the event that an unexpected unfortunate situation occurs as a result, a ground for the validity of the medical practice can be provided.

With respect to the check log document, it is preferred to place a link serving as an independent related document from the instruction document to the check log document. In some cases, the check log document may be inserted into the end of the instruction document.

While an embodiment of the present invention has been described, the specific configuration of the present invention is not limited to the embodiment. Design changes and the like in the embodiment are included in the present invention without departing from the spirit and scope of the invention.

For example, while the present invention has been described using the instruction document on medical care as an example, the present invention is also useful in typical transaction slips, contract terms, and the like, which are often required to be checked according to many rules beforehand and afterward.

## Claims

1. An instruction document creating system for creating an instruction document mainly in a medical field, comprising:
(i) instruction item-specific rules recording means configured to record rules to be observed with respect to each of instruction items in the instruction document;
(ii) rule consistency check means configured to perform checks on consistency between the instruction items in the instruction document and the rules recorded in the instruction item-specific rules recording means on the basis of the rules; and
(iii) check log document creating means configured to create results of the rule consistency checks as a document related to the instruction document.

2. The instruction document creating system of claim 1, wherein the rule consistency check means comprises information-required-to-check-consistency request means configured to request information required to check the consistency from medical record documents of a patient involved.

3. The instruction document creating system of claim 1 or 2, wherein the rule consistency check means comprises after-consistency checks additional information write means configured to request a write, to the medical record documents of the patient involved, of an additional disease name, an additional finding, and an additional instruction document that have occurred in a process of the consistency checks.

4. The instruction document creating system of any one of claims claim 1 to 3, wherein the rule consistency check means comprises after-consistency confirmation additional information write means configured to, after an additional disease name, an additional finding, and an additional instruction document that have not been confirmed in a process of the consistency checks are confirmed, request a write of the additional disease name, the additional finding, and the additional instruction document to the medical record documents of the patient involved.

5. The instruction document creating system of any one of claims 1 to 4, wherein the instruction item-specific rules recording means comprises multiple instruction items-specific rules recording means configured to record rules corresponding to multiple instruction items together.
